Europäisches Patentamt

European Patent Office

Office européen des brevets

(19)

(11) Publication number: **0 301 489 B1**

# EUROPEAN PATENT SPECIFICATION

(12)

(45) Date of publication of patent specification: **26.08.92**   (51) Int. Cl.⁵: **A61F 5/02**

(21) Application number: **88112067.9**

(22) Date of filing: **26.07.88**

(54) **Surgically implantable device for spinal columns.**

(30) Priority: **29.07.87 US 79457**
**28.08.87 US 90522**

(43) Date of publication of application:
**01.02.89 Bulletin 89/05**

(45) Publication of the grant of the patent:
**26.08.92 Bulletin 92/35**

(84) Designated Contracting States:
**CH DE ES FR GB LI NL**

(56) References cited:
**US-A- 3 242 922**
**US-A- 4 611 581**
**US-A- 4 648 388**
**US-A- 4 655 199**

(73) Proprietor: **ACROMED CORPORATION**
**3303 Carnegie Avenue**
**Cleveland Ohio 44115(US)**

(72) Inventor: **Heinig, Charles F.**
**3521 Johnny Cake Lane**
**Charlotte, NC 28226(US)**
Inventor: **Asher, Marc A.**
**4017 D. West 84th St.**
**Prairie Village, Kansas(US)**
Inventor: **Strippgen, Walter E.**
**4960 McIntyre**
**Golden, Colorado 80403(US)**
Inventor: **Weinstein, James N.**
**1250 Melrose Avenue**
**Iowa City, Iowa 52240(US)**

(74) Representative: **Rottmann, Maximilian R.**
**c/o Rottmann, Maspoli + Zimmermann AG**
**Glattalstrasse 37**
**CH-8052 Zürich(CH)**

Rank Xerox (UK) Business Services

EP 0 301 489 B1

# Description

## Background of the Invention

### Field of the Invention

The present invention relates to the surgical correction of spinal columns. Particularly, the present invention is a surgically implantable device connectable with spinal bodies of the spinal column for maintaining the relative positions of the spinal bodies.

### Description of the Prior Art

It is known that the human spinal column is prone to deformation and degeneration. Deformation includes abnormal curvature of the spinal column, such as lordosis, kyphosis or scoliosis. Degeneration includes injury to, or failure of, the disc and ligaments located between adjacent spinal bodies of the spinal column. These conditions often require surgery to correct. During the surgical procedure, implantable devices are connected with the spinal bodies of the spinal column to correct the condition and to maintain the spinal bodies in a desired predetermined relationship.

Surgically implantable devices for the correction of spinal columns are known. U.S. Patent No. 4,611,581 discloses one such implantable device in which a relatively rigid elongated plate is connectable with spinal bodies of the spinal column to maintain the spinal bodies in a desired relationship. However, the relatively rigid plate is not, as a practical matter, bendable to conform to a desired curvature of the spinal column. Furthermore, the bulk of the plate may preclude its use on some areas of the spinal column.

U.S. Patent No. 4,648,388 discloses another type of implantable device in which a rod which is deformed by a surgeon to conform to a desired curvature of a spinal column. The rod is connected with spinal bodies of the spinal column to maintain the spinal bodies in a desired relationship and having a desired curvature.

U.S. Patent No. 3,242,922 discloses another implantable device including a pair of plates which clamp the spinous processes of spinal bodies therebetween. The device also includes a pair of adjustable length rods. Each of the rods is pivotably connected at one end to a respective one of the plates. The other end of each of the rods is connectable with a patient's sacrum.

## Summary of the Invention

The present invention is a surgically implantable device for correcting and maintaining the relationship of spinal bodies of a spinal column. The surgically implantable device of the present invention provides a relatively rigid plate having at least one bendable rod fixedly connected to the plate and extending therefrom. The plate corrects and maintains the lateral relationship of spinal bodies and the rod is deformable to a desired curvature to maintain other spinal bodies in a desired lateral and dorsal relationship.

The plate has a first major side surface for facing the first spinal body and a second major side surface. The plate has at least one opening extending through the first and second major side surfaces for receiving a fastener to connect the plate with the first spinal body. A first rod is fixedly connected to the plate and extends therefrom. The first rod is bendable to conform to a desired curvature of a portion of the spinal column. The first rod has a part for connection with a second spinal body.

The plate has a longitudinal central axis. The opening in the plate may be an elongated slot which has a longitudinal central axis extending in a direction parallel to the longitudinal central axis of the plate. The first rod extends from the plate in a direction parallel to the longitudinal central axis of the plate.

In one embodiment of the present invention, the first rod is offset from the longitudinal central axis of the plate. The surgically implantable device may be reversed so that the second major side surface of the plate faces the spinal bodies and the rod is displaced in the lateral direction. Thus, the surgeon has greater freedom in locating the fastener in the spinal body.

In another embodiment of the present invention, a second rod is fixedly connected to the plate and extends therefrom. The second rod extends in a direction parallel to the longitudinal central axis of the plate and opposite to the first rod. The second rod has a part for connection with a third spinal body. The second rod is also deformable to a desired curvature of a portion of the spinal column.

The first and second rods are fixedly connected to the plate at respective connecting portions. The connecting portions have relatively large blend radii extending between the respective rod and the plate for minimizing stress loading in the connecting portions.

## Brief Description of the Drawings

Further features of the present invention will become apparent to those skilled in the art to which the present invention relates from reading the following specification with reference to the accompanying drawings, in which:
Fig. 1 is a view of a first embodiment of the surgically implantable device, according to the present invention, connected to spinal bodies of a spinal column;

Fig. 2 is a view of the device in Fig. 1, taken approximately along line 2-2 in Fig. 1;

Fig. 3 is an enlarged plan view of the device in Fig. 1;

Fig. 4 is a cross sectional view of the device of Fig. 3 taken approximately along line 4-4 in Fig. 3;

Fig. 5 is an enlarged cross sectional view of a portion of the device in Fig. 1 connected with a spinal body;

Figs. 6 and 7 are perspective views of clamps for connecting a rod of the device with a spinal body;

Fig. 8 is an enlarged plan view of a second embodiment of the device of the present invention;

Fig. 9 is a cross sectional view of the device in Fig. 8 taken approximately along line 9-9 in Fig. 8;

Fig. 10 is a schematic view of spinal bodies prepared for receiving the device of the present invention;

Fig. 11 is a schematic view of the device of Fig. 8 connected with spinal bodies of the spinal column;

Figs. 12 and 13 are views similar to Figs. 1 and 2, of a third embodiment of the device of the present invention connected with spinal bodies of the spinal column;

Fig. 14 is an enlarged plan view of the device in Figs. 12 and 13; and

Fig. 15 is a cross sectional view of the device in Fig. 14 taken approximately along line 15-15 in Fig. 14.

Fig. 16 is a view of a fourth embodiment of the surgically implantable device, according to the present invention, connected with spinal bodies of a spinal column;

Fig. 17 is a view of the device in Fig. 16, taken approximtely along line 17-17 of Fig. 16;

Fig. 18 is an enlarged plan view of a portion of the device of Fig. 16;

Fig. 19 is an cross sectional view of the device in Fig. 18, taken approximately along line 19-19 of Fig. 18;

Fig. 20 is an enlarged cross sectional view of a portion of the device of Fig. 16 connected with a spinal body;

Figs. 21 and 22 are views, similar to Figs. 16 and 17, illustrating a surgically implantable device according to a fifth embodiment of the present invention;

Fig. 23 is an enlarged plan view of a portion of the device of Fig. 21; and

Fig. 24 is an elevational view of the device of Fig. 23, taken along line 24-24 of Fig. 23.

Description of Preferred Embodiments

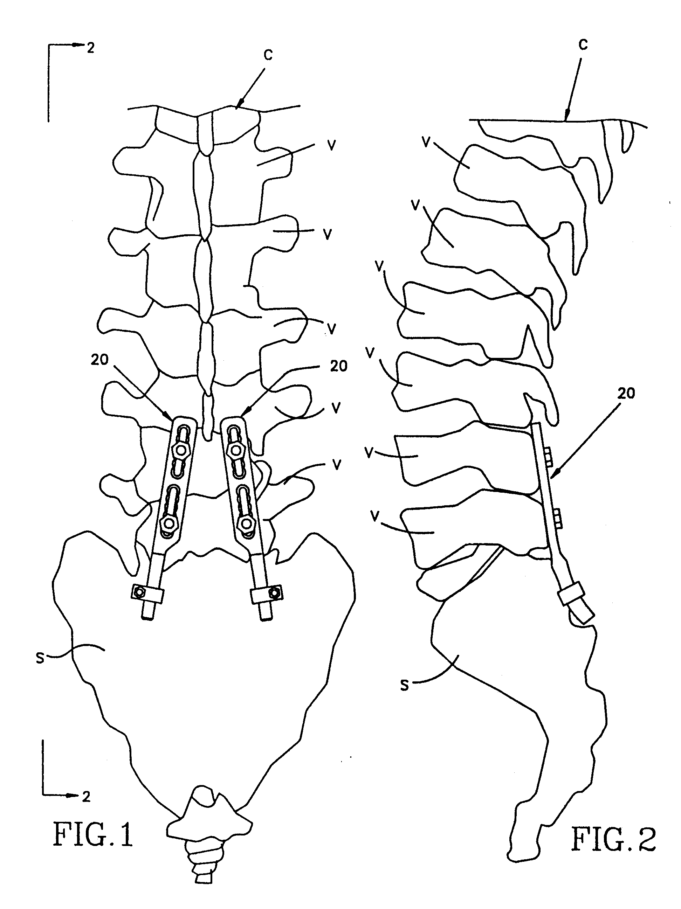

A pair of surgically implantable devices 20 (Figs. 1 and 2), according to a first embodiment of the present invention, are connected with spinal bodies, such as a sacrum S and vertebrae V which make up a spinal column C. Each of the surgically implantable devices 20 is for correcting deformation and/or degeneration of the spinal column C and for maintaining the spinal bodies V, S in desired relative positions. The surgically implantable devices 20 are illustrated by way of example as being connected to lumbar vertebrae and the sacrum of the spinal column C. However, it will be apparent that the surgically implantable devices 20 may be used in other regions of the spinal column C, such as on the thoracic and/or cervical vertebrae. It will also be apparent that a single surgically implantable device 20 may be used to maintain the relative positions of the spinal bodies V, S.

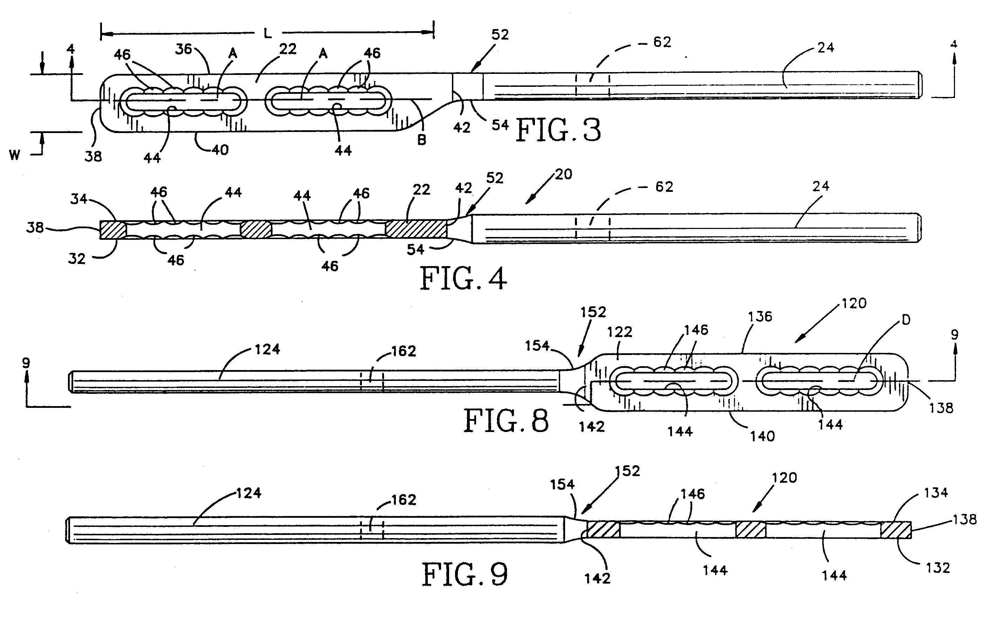

The surgically implantable device 20 (Figs. 3 and 4) includes a plate 22 and a rod 24. The plate 22 is connectable with at least one spinal body V, as illustrated in Fig. 1. The plate 22 has a first major side surface 32 (Fig. 4) for facing the spinal bodies V when the plate is connected with a spinal body. The plate 22 also has a second major side surface 34 which extends in a direction generally parallel to the first major side surface 32. The plate 22 of the surgically implantable device 20 is elongated. That is, the major side surfaces 32, 34 have a length L which is larger than their width W. The major side surfaces 32, 34 are connected by a plurality of minor side surfaces 36, 38, 40, 42.

The plate 22 has a pair of openings or elongated slots 44 extending through the first and second major side surfaces 32, 34. The slots 44 receive a fastener 48 (Fig. 5) to connect the plate 22 with the spinal body V. The slots 44 provide for some adjustment or relocation of the plate 22 along the fastener 48 relative to the spinal bodies V during the surgical procedure. Each of the slots 44 (Fig. 3) have a longitudinal central axis A which extends in a direction parallel to the longitudinal central axis B of the plate 22, and preferably coaxially therewith. A plurality of recesses 46 are spaced along the length of each slot 44. The recesses 46 are located in both the first and second major side surfaces 32, 34 to permit the plate 22 to be reversed, or turned over, so that the second major side surface 34 faces the spinal bodies, as is described below.

The fastener 48 (Fig. 5) includes a screw 49 and a nut 50. The screw 49 has a first externally threaded portion 49a for threaded engagement with a surface defining an opening 51 in the spinal body V to connect the screw with the spinal body. The screw 49 also has a second externally threaded portion 49b for threaded engagement with the nut 50. When the nut 50 is threaded onto the second threaded portion 49b of the screw 49, it is drawn against the plate 22 of the surgically implantable device 20 to connect the plate with the spinal body V. One of the recesses 46 receives a portion 50a of the nut 50 to block sliding movement of the plate 22 relative to the screw 49 and, thus, the spinal body V.

Each of the recesses 46 is preferably frustoconical to match a frustoconical portion 50a of the nut 50 (Fig. 5). When the portion 50a of the nut 50 is seated in a recess 46, the plate 22 is prevented from sliding relative to the screw 49 when the screw 49 is connected with the spinal body V and the nut 50 is tightened against the plate. A spacer device, such as another nut 50b, is used on the screw 49 to space the plate 22 away from the spinal body V. While one type of fastener 48 is illustrated in Fig. 5, it will be apparent that other types of fasteners may be used.

The rod 24 of the surgically implantable device 20 is fixedly connected to the plate 22. The rod 24 extends from the minor side surface 42 of the plate 22. The rod 24 is connectable with the spinal body or sacrum S (Fig. 1) of the spinal column C. While the rod 24 is illustrated as connected to the sacrum S, it will be apparent that the rod could be located so it is connected with one of the vertebrae V.

The rod 24 has an elongated cylindrical configuration. The rod 24 is bendable or permanently deformable to conform to a desired curvature of the spinal column C. A hydraulic press located in the operating room is preferably used to deform the rod 24 to the desired curvature. The rod 24 may be deformed to incorporate a compound bend. That is, when the surgically implantable device 20 is connected with the spinal column C, the rod 24 is bent in the dorsal and the lateral planes. The plate 22 may be connected with the spinal column C before the rod 24 is connected therewith. Thus, the rod 24 can be restrained from moving by the plate 22 to aid installation. After the rod 24 is deformed, it is still rigid enough to maintain the spinal bodies V, S in a relationship which has a desired curvature.

A part 62 of the rod 24 is for connection with the spinal body S. Figs. 6 and 7 illustrate clamps 64, 66, respectively, for connecting the part 62 of the rod 24 with the spinal body S. Fig. 6 illustrates a one-piece clamp 64 having a clamp portion 74 with an opening 76 which circumscribes and engages the part 62 of the rod 24. The part 62 of the rod 24 preferably has a knurled or roughened exterior surface.

A screw portion 84 of a fastener 82 is inserted through another opening 86 extending through leg portions 88 projecting from the clamp portion 74 and offset therefrom. The screw 84 is threaded into an opening in the spinal body S. The opening 86 extends in a direction generally perpendicular to the opening 76. When a nut 90 of the fastener 82 is tightened against the clamp 64, the leg portions 88 are drawn together and the clamp portion 74 grippingly engages the outer circumference of the part 62 of the rod 24 to connect the rod with the spinal body S. A second nut 90a is used to space the clamp 64 away from the spinal body V or S.

A two-piece clamp 66 is illustrated in Fig. 7. The clamp 66 has a pair of clamp halves 92, 94. Each of the clamp halves 92, 94 has a clamp portion 96 and a leg portion 98 extending therefrom. The clamp portions 96 define an opening 100 for circumscribing the part 62 of the rod 24. The part 62 of the rod 24 has a transverse slot 102 for receiving a nib 104 on the clamp half 94 to prevent rotational and axial movement of the rod relative to the clamp 66. The screw 84 of the fastener 82 is received in openings 106 in the leg portion 98 to connect the clamp 66 with the spinal body S. In Fig. 1, it will be seen that the fasteners 82 are located to the outside of the rods 24. This is merely for illustration purposes and it will be apparent that the surgeon may elect to locate the fasteners on the inside of the rods 24 for better securement to the sacrum S.

The rod 24, in the first embodiment of the present invention, is offset from the longitudinal central axis B of the plate 22. Thus, the surgeon may reverse, or turn over, the surgically implantable device 20 for optimal placement of the fasteners 48, 82 in the spinal bodies V, S. Since the plate 22 has recesses 46 in both major side surfaces 32, 34, the plate is blocked from sliding movement relative to the fastener 48. The end of rod 24 extending away from the plate 22 may be trimmed if necessary as may the ends of the screws 49, 84 extending beyond their respective nuts 50, 90.

The rod 24 and plate 22 are interconnected at a connecting portion 52. The connecting portion 52 has relatively large blend radii 54 extending between the connecting portion 52 and the plate 22, and between the connecting portion 52 and the rod 24. The relatively large blend radii 54 assure that stress loading in the connecting portion 52 is minimized.

Figs. 8 and 9 illustrate a second embodiment of a surgically implantable device 120, according to the present invention. The surgically implantable device 120 is similar to the surgically implantable device 20 illustrated in Figs. 3 and 4. The surgically implantable device 120 includes an elongated plate 122 and a rod 124. The plate 122 is connectable with a spinal body V or S. The plate 122 has first and second major side surfaces 132, 134, respectively, which extend generally parallel. The major side sufaces 132, 134 are interconnected by a plurality of minor side surfaces 136, 138, 140, 142.

The plate 122 includes a pair of longitudinally extending elongated slots 144. Recesses 146 are spaced along the slots 144 in only the second major side surface 134 of the plate 122 which faces away from the spinal bodies V, S of the spinal column C when the device 120 is connected with the spinal column. The rod 124 is fixedly connected to and extends from the minor side surface 142 of the plate.

The rod 124 extends from a central portion of the minor side surface 142 and is preferably coaxial with the longitudinal central axis D of the plate 122. The plate 122 of the second embodiment may be connected with a spinal body V or S of the spinal column C so the rod 124 disposed toward the sacrum S or the rod is disposed away from the sacrum, relative to the plate. The rod 124 has a part 162 which is connectable with the spinal body V or S.

The plate 122 and the rod 124 are interconnected at a connecting portion 152. The connecting portion 152 has relatively large blend radii 154 connecting the plate 122 with the connecting portion 152, and the connecting portion 152 with the rod 124. Thus, stress loading in the connecting portion 152 is minimized.

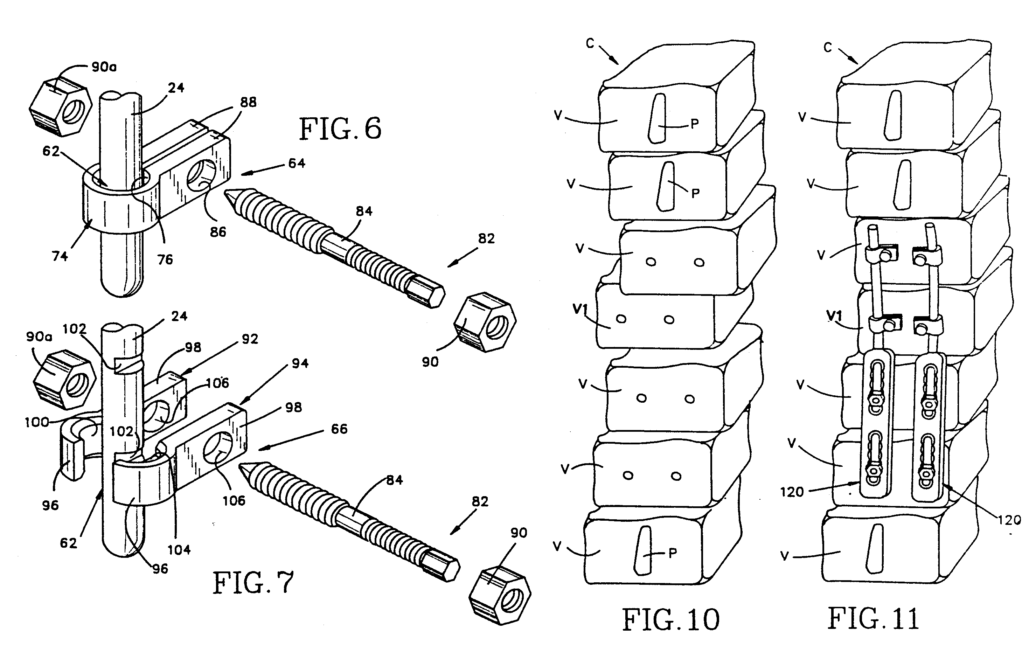

The spinal bodies V are schematically illustrated in Fig. 10 as blocks which have been prepared for receiving the surgically implantable device 120. The spinal bodies V which are to receive the device 120 have had the spinous process P removed. However, it will be apparent that the spinous process P may not have to be removed if the installation of the surgically implantable device 120 will not be affected by the spinous process. One of the spinal bodies $V_1$ is illustrated as being displaced laterally and dorsally from a normal or desired position in the spinal column C. It is this displacement that the present invention seeks to correct.

Fig. 11 illustrates the surgically implantable device 120, according to the second embodiment of the present invention, connected with spinal bodies V of the spinal column C. The rod 124 of the surgically implantable device 120 extends upwardly along the spinal column C, as viewed in Fig. 11, from the plate 122. The rod 124 has been bent by the surgeon during the surgical procedure in the dorsal plane to a desired curvature of the spinal column C. It will be apparent that the rod 124 could also be bent in the lateral plane as well. The clamps and fasteners used to connect the surgically implantable device 120 with the spinal bodies V are similar to those described above in the first embodiment and will not be described further.

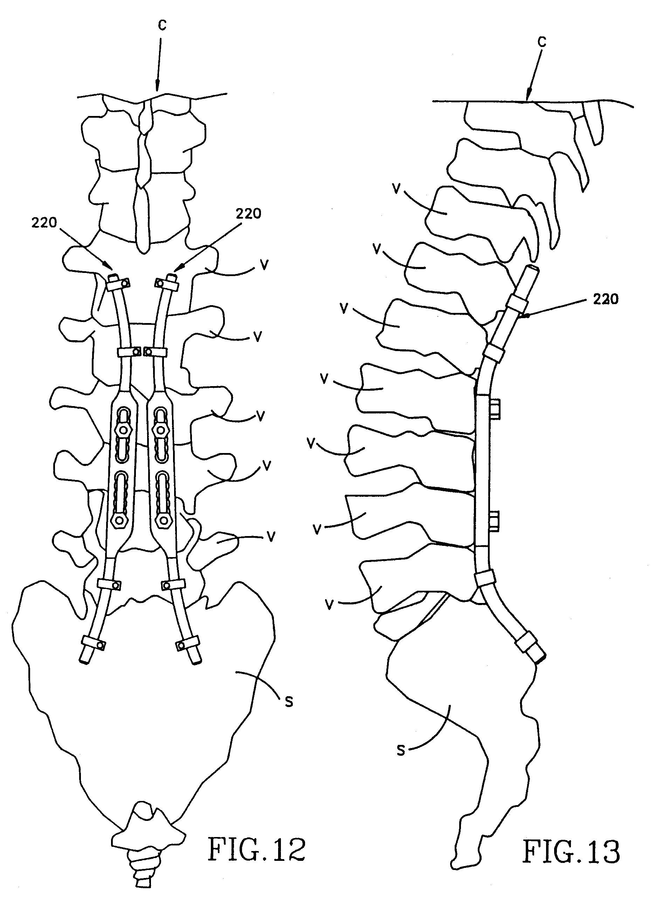

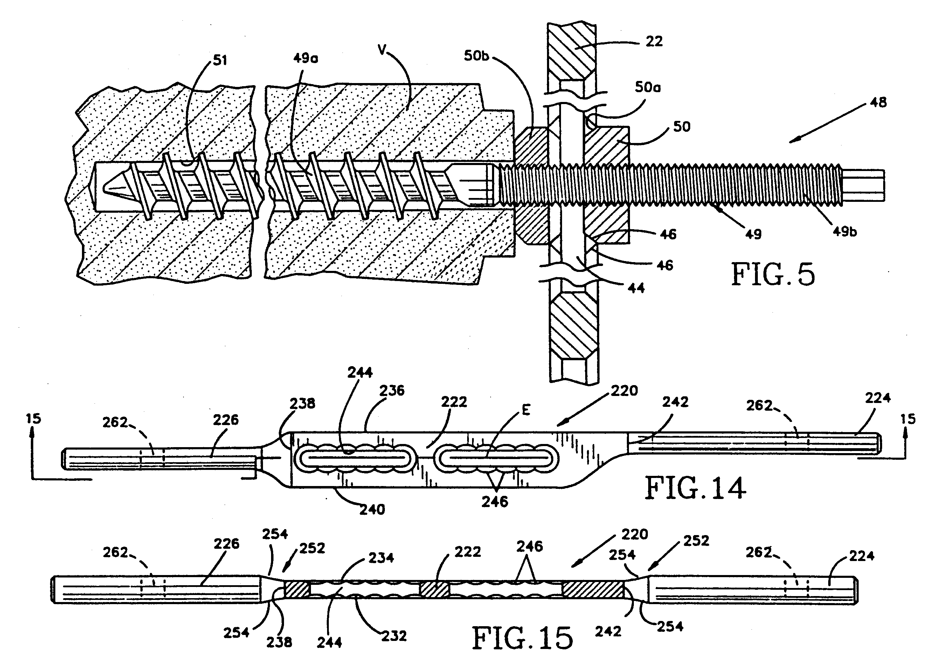

A third embodiment of the present invention is illustrated in Figs. 12 and 13. A pair of surgically implantable devices 220 are connected with spinal bodies V, S of the spinal column C. The surgically implantable device 220 (Figs. 14 and 15) includes a plate 222 and a pair of rods 224 and 226 fixedly connected to the plate. The rods 224, 226 extend generally parallel and in opposite directions from the plate 222. The rods 224, 226 extend in a direction parallel to the longitudinal central axis E of the plate 222. The plate 222 has major side surfaces 232, 234 interconnected by minor side surfaces 236, 238, 240, 242. The rod 224 extends from the minor side surface 242 of the plate 222. The rod 226 extends from the minor side surface 238. Each of the rods 224, 226 has a part 262 for connection with a spinal body V or S.

The plate 222 has a pair of elongated slots 244 extending through the major side surfaces 232, 234. The slots 244 have recesses 246 spaced therealong in both of the major side surfaces 232, 234. Thus, the surgeon may reverse the surgically implantable device 220 on the spinal column C so that fasteners connecting clamps with the rods 224, 226 are located optimally in a respective spinal body V, S.

The rod 224 is offset from the longitudinal central axis E of the plate 222. The rod 226 is located along the longitudinal central axis E of the plate 222. It will be obvious that both rods 224, 226 could be offset or both rods disposed along the longitudinal central axis E of the plate 222. The exact configuration and location of the rods 224, 226 will be dictated by the patient's requirements and selected by the surgeon during the preparation for the surgical procedure. The rods 224, 226 are deformable to conform to a desire curvature of a portion of the spinal column. The rods 224, 226 are illustrated in Figs. 12 and 13 as having compound bends. That is, the rods are bent in the lateral and dorsal planes when the surgically implantable device 220 is connected with the spinal column C.

The plate 222 and the rods 224, 226 are interconnected at connecting portions 252. The connecting portions 252 have relatively large blend radii 254 connecting the plate 222 with one of the connecting portions 252, and the connecting portions 252 with the respective rods 224, 226. Thus, stress loading in the connecting portions 252 is minimized.

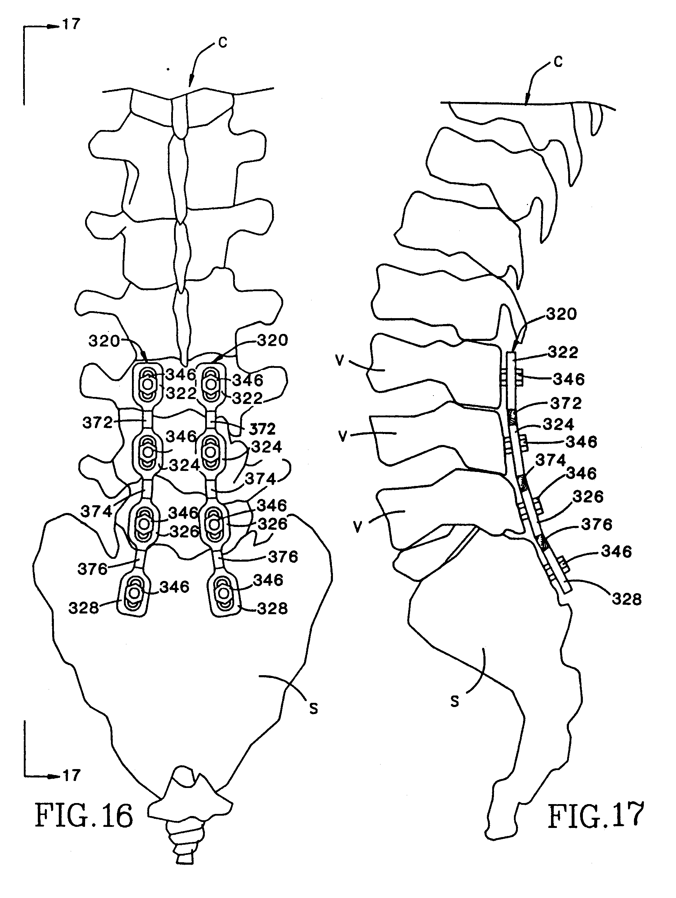

A pair of surgically implantable devices 320 (Figs. 16 and 17), according to a fourth embodiment of the present invention, are connected with spinal bodies, such as a sacrum S and vertebrae V which make up a portion of the spinal column C. Each of the surgically implantable devices 320 is for correcting deformation and/or degeneration of the spinal column C and for maintaining the spinal bodies in desired relative positions. It will be apparent that a single surgically implantable device 320 may be used to maintain the relative positions of the spinal bodies V, S of the spinal column C dependent upon the amount of correction required.

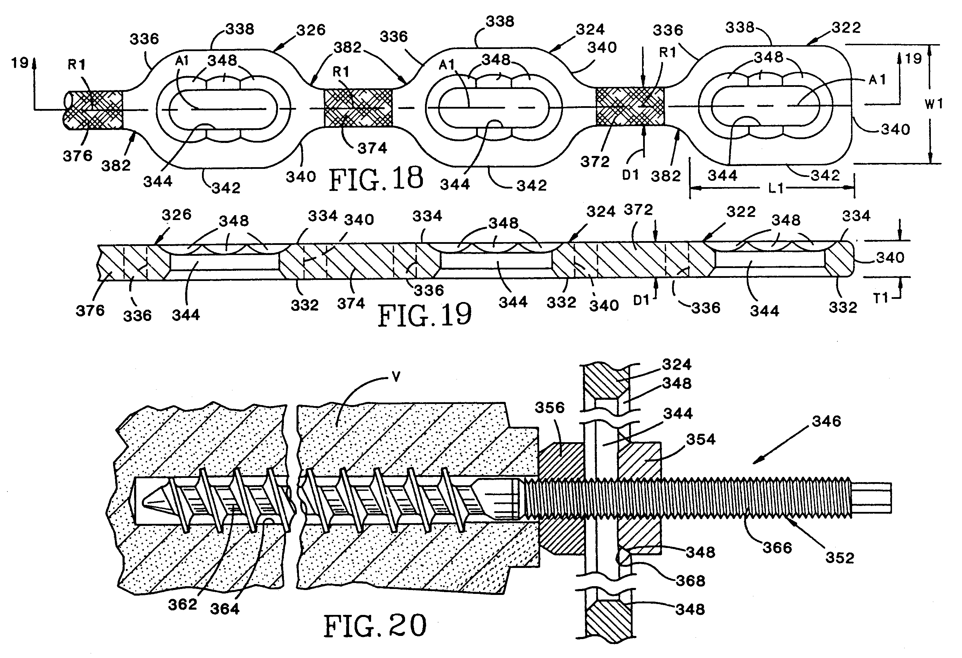

Each surgically implantable device 320 includes a plurality of identical plates 322, 324, 326, 328. Each of the plates 322, 324, 326, 328 is connectable with a separate one of the spinal bodies V, S. Each of the plates 322, 324, 326, 328 (only three of which are illustrated in Figs. 18 and 19, but are representative of all the plates) has a first major side surface 332 for facing the spinal body V or S when the plate is connected with the spinal body. Each of the plates 322, 324, 326, 328 also has a second major side surface 334 which extends in a direction generally parallel to the first major side surface 332.

Each of the plates 322, 324, 326, 328 has an elongate and generally rectangular configuration. That is, the major side surfaces 332, 334 of each of the plates 322, 324, 326, 328 have a length L1 which is larger than its width W1. Each of the plates 322, 324, 326, 328 also have a thickness T1 which is less than the width W1. The major side surfaces 332, 334 of each of the plates 322, 324, 326, 328 are connected by a plurality of minor side surfaces 336, 338, 340, 342.

Each of the plates 322, 324, 326, 328 has an elongate opening or slot 344 extending through the first and second major side surfaces 332, 334. Each of the slots 344 receive a fastener 346 (Fig. 20) to connect each of the plates 322, 324, 326, 328 with a separate one of the spinal bodies V, S. The slots 344 permit adjustment or relocation of the device 320 along the spinal column C relative to the fasteners 346 and allow greater flexibility in locating the fasteners during the surgical procedure. Each of the slots 344 has a longitudinal central axis which extends in a direction parallel to the longitudinal central axis A1 of the respective plates 322, 324, 326, 328 and preferably coaxially therewith. A plurality of recesses 348 are spaced along the length of each slot 344.

Each of the devices 320 is connected with the spinal bodies V, S of the spinal column C by a plurality of the fasteners 346. The devices 320 and fasteners 346 are made from materials compatible with human tissue. Each of the fasteners 346 includes a screw 352 and a pair of nuts 354, 356. The screw 352 has a first externally threaded portion 362 for threaded engagement with a surface defining an opening 364 in the spinal body V to connect the screw with the spinal body. The screw 352 also has a second externally threaded portion 366 for threaded engagement with the nuts 354, 356. The nut 356 spaces the plate 324 away from the spinal body V. The nut 354 is threaded onto the second threaded portion 366 of the screw 352 and is tightened against the plate 324 opposite the nut 356 to connect the plate with the spinal body V. The end portion of the screw 352 which extends beyond the nut 354 may be trimmed off.

The nut 354 has a frustoconical portion 368 facing the spinal body V which is received in one of the recesses 348 to block sliding movement of the plate 324 relative to the screw 352 and thus relative to the spinal body V. Each of the recesses 348 is preferably frustoconical to closely match the frustoconical portion 368 of the nut 354. While the fastener 346 is illustrated in Fig. 20, it will be apparent that other types of fasteners may be used.

Each of the surgically implantable devices 320 also includes means, separate from the fasteners 346, for interconnecting the plates 322, 324, 326, 328. The means for interconnecting the plates 322, 324, 326, 328 consists essentially of rods 372, 374, 376 (Figs. 16 and 17). The rod 372 connects the plate 322 with the plate 324. The rod 374 connects the plate 324 with the plate 326. The rod 376 connects the plate 326 with the plate 328. Each of the rods 372, 374, 376 (Figs. 18 and 19) has a circular cross section extending in a plane perpendicular to the longitudinal central axis R1 of each of the rods. The diameter D1 of each of the rods 372, 374, 376 is less than the width W1 of the plates 322, 324, 326, 328.

The rod 372 is fixedly connected at a first end portion to the minor side surface 336 of the plate 322. The rod 372 is also fixedly connected with the minor side surface 340 of the plate 324. The rod 374 is fixedly connected at a first end portion to the minor side surface 336 of the plate 324. The rod 374 is also fixedly connected with the minor side surface 340 of the plate 326. The rod 376 is fixedly connected at a first end portion to the minor side surface 336 of the plate 326. The rod 376 is also fixedly connected with the minor side surface 340 of the plate 328.

Each of the rods 372, 374, 376 is bendable or permanently deformable to position the plates 322, 324, 326, 328 in a desired location relative to one another to position the spinal bodies S, V to conform to a desired curvature of the spinal column C. A suitable press located in the operating room is preferably used to bend the rods 372, 374, 376 during the surgical procedure. The rods 372, 374, 376 are bendable in plural directions. For example, each of the rods 372, 374, 376 is bendable in at least two perpendicular planes extending in a direction parallel to the longitudinal central axis R1 of the rods. For example, the surgically implantable devices 320 have the rods 372, 374, 376 bent in the lateral or sagittal plane, as illustrated in Fig. 16, and in the dorsal plane, as illustrated in Fig. 17. After the rods 372, 374, 376 are bent, they still have sufficient rigidity to maintain the spinal bodies V, S in the desired relative positions to maintain the desired curvature of the spinal column C.

Each of the rods 372, 374, 376 is connected with their respective plates 322, 324, 326, 328 at a connecting portion 382. The connecting portions 382 have relatively large blend radii extending between the rods 372, 374, 376 and the plates 322, 324, 326, 328. The relatively large blend radii serve to minimize stress loading in the connecting portions 382.

While each device 320 is illustrated as having four plates 322, 324, 326, 328 and three rods 372, 374, 376, it will be apparent that other numbers of plates and rods can be used dependent upon the needs of the patient. It will also be apparent that the devices 320 may be connected to the anterior portions of the spinal bodies V.

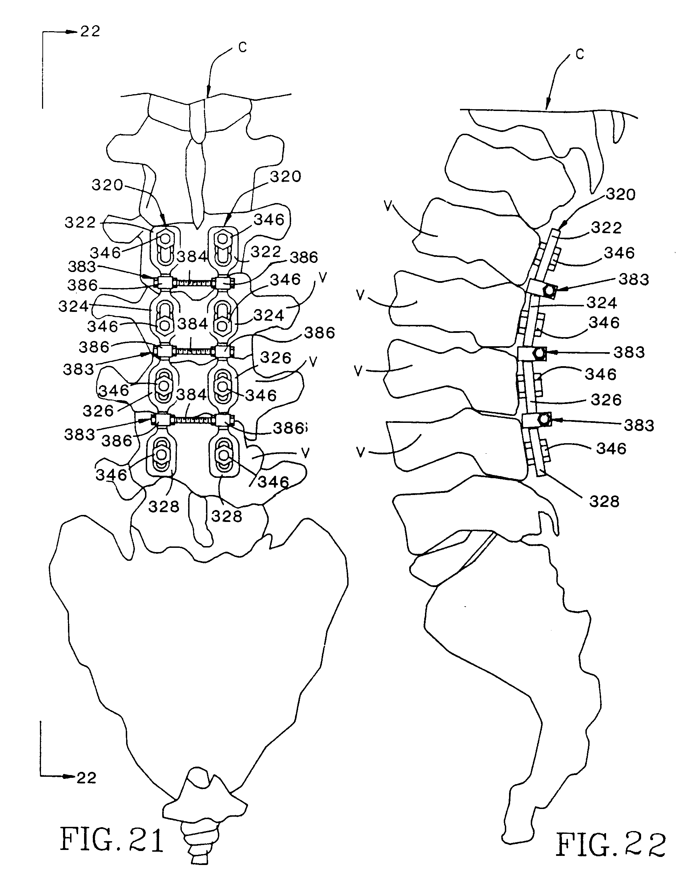

A fifth embodiment of the present invention is illustrated in Figs. 21 and 22. The fifth embodiment includes a pair of surgically implantable devices 320, which are identical to those described above and illustrated in Figs. 18 and 19. The pair of surgically implantable devices 320 are connected with spinal bodies V by fasteners 346 in a similar manner to that described above for the fourth embodient. The devices 320 are shown connected with a portion of the spinal column C different than that which is illustrated in Figs. 16 and 17.

The fifth embodiment of the present invention further includes a plurality of identical bridges 383. Each of the bridges 383 connects the pair of surgically implantable devices 320 together. The bridges 383 add rigidity to the assembly and prevents the pair of surgically implantable devices 320 from moving relative to one another. Thus, a very rigid structure is connected with the spinal bodies V to maintain the spinal bodies in desired relative positions.

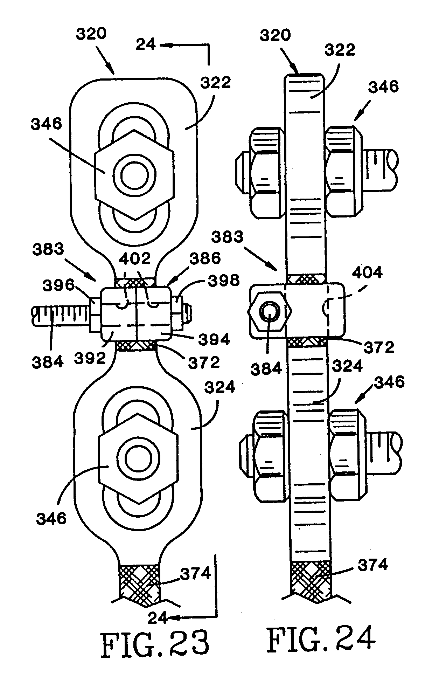

Each of the bridges 383 includes a threaded rod 384 and a pair of clamps 386. Each of the pair of clamps 386 are disposed at axially opposite end portions of the threaded rod 384. Each of the pair of clamps 386 (Figs. 23 and 24) includes a pair of identical clamp halves 392, 394 and a pair of nuts 396, 398. The clamp halves 392, 394 each have an opening 402 through which a portion of the threaded rod 384 is received and a semi-cylindrical surface which, when the clamp halves abut, form another opening 404 which receives one of the rods 372, 374, 376.

During the surgical procedure once the devices 320 have been implanted, the nut 396 is threaded onto an intermediate portion of the threaded rod 384. The inner clamp half 392 then receives the threaded rod 384 in the opening 402. The inner clamp half 392 is then positioned adjacent the rod 372 so that the semi-cylindrical surface defining a portion of the opening 404 circumscribes a portion of the rod 372. The rod 372 is knurled on its exterior surface to provide a suitable surface to be clamped. The nut 396 is then threaded towards the end portion of a threaded rod 384 to engage the inner clamp half 392.

The clamp half 394 receives the threaded rod portion 384 in the opening 402. The clamp half 394 is slid into engagement with the clamp half 392 so the other semi-cylindrical surface defining the opening 404 circumscribes another portion of the rod 372. The nut 398 is then threaded onto the end portion of the threaded rod 384. The nut 398 is tightened so that the clamp 386 exerts a clamping or gripping force about the rod 372. The clamp 386 at the axially opposite end portion of the threaded rod 384 is installed in a similar manner to rigidly connect the surgically implantable devices 320 together as illustrated in Fig. 21. The end portions of the threaded rod 384 which extend beyond the outermost nuts may then be trimmed away. It will be apparent that any number of bridges 383 may be used as is deemed necessary by the surgeon.

## Claims

1. An apparatus (20, 120, 220) for maintaining the relative positions of spinal bodies of a spinal column, said apparatus comprising:

   a plate (22, 122, 222) for connection with a first spinal body, said plate having a central axis (A, D, E) and having first and second major side surfaces (32, 34, 132, 134, 232, 234), one of said first and second major side surfaces being provided for facing the first spinal body, said first and second major side surfaces extending substantially parallel to each other, and being interconnected by a plurality of minor side surfaces (36, 38, 40, 42, 136, 138, 140, 142, 236, 238, 240, 242) extending between said first and second major side surfaces;

   surface means defining an opening (44, 144, 244) extending through said plate between said first and second major side surfaces for receiving a fastener (48) to connect said plate with the first spinal body, characterized by

   a first rod (24, 124, 224) fixedly and non-releasably connected to and extending from one of said plurality of minor side surfaces of said plate and being connectable with a second spinal body, said first rod being bendable for the purpose of conforming to a desired curvature of a portion of the spinal column; and

a connecting portion (52, 152, 252) located between said first rod and said one minor side surface and having a transition zone of gradually changing cross section for minimizing stress loading in said connecting portion due to bending of said first rod.

2. The apparatus according to claim 1, characterized in that it further includes a second rod (226) fixedly connected to and extending from another minor side surface of said plate in a direction parallel to the central axis of said plate and opposite to said first rod, said second rod being connectable with a third spinal body and being bendable for the purpose of conforming to a desired curvature of another portion of the spinal column.

3. The apparatus according to claim 1, characterized in that said first rod has a longitudinal central axis which is spaced apart from and extends parallel with the central axis of said plate, and wherein one of said major side surfaces is for facing the first spinal body.

4. The apparatus according to claim 1, characterized in that one of said major side surfaces is for facing the first spinal body, and wherein said plate is elongate and the opening in said plate is elongate and extends in a direction substantially parallel to the central axis of said plate and further includes first surface means defining a plurality of recesses (46, 146, 246) in said first major side surface of said plate spaced along the length of the opening and second surface means defining a second plurality of recesses (46, 146, 246) in said second major side surface of said plate spaced along the length of the opening, one recess of said first and second plurality of recesses for receiving a portion of the fastener to block sliding movement of said plate relative to the fastener.

5. The apparatus according to claim 1, characterized in that said first rod has a longitudinal central axis (R1) which extends coaxially with the central axis of said plate.

6. The apparatus according to claim 1, characterized in that said plate is elongate and the opening in said plate is elongate and extends in a direction substantially parallel to the central axis of said plate and further including surface means defining a plurality of recesses (46, 146) in said second major side surface of said plate spaced along the length of the opening, one of said plurality of recesses for receiving a portion of the fastener to block sliding movement of said plate relative to the fastener.

7. The apparatus according to claim 1, characterized in that it further includes clamp means (64, 66) for connecting said first rod with the second spinal body, said clamp means comprising a member having surfaces circumscribing a portion of said first rod and a leg extending from said member, said leg having an opening offset from said member for receiving another fastener to connect said clamp means to the second spinal body.

8. An apparatus (320) for maintaining the relative positions of spinal bodies of a spinal column, characterized in that said apparatus comprises

a pair of plates (322, 324) which are spaced apart a predetermined distance, each of said pair of plates being connectable with a separate one of the spinal bodies;

each of said pair of plates having a surface (332, 334) defining an opening (344) which extends through said plate for receiving a fastener (346) for extension into the spinal body to connect said plate with the spinal body;

means independent of the fastener for interconnecting said pair of plates and consisting essentially of a rod (372) fixedly and non-releasably connected to each of said pair of plates, said rod having a length no greater than said predetermined distance, said rod being bendable in plural directions to locate each of said pair of plates adjacent the respective spinal body.

9. The apparatus according to claim 8, characterized in that each of said pair of plates has a thickness (T1) taken in a direction substantially parallel to the direction that the opening extends through said plate, said thickness being of a dimension less than a width (W1) of each of said pair of plates taken substantially perpendicular to the thickness of a respective one of said pair of plates, and wherein said rod has a circular cross section taken in a plane perpendicular to the longitudinal central axis (R1) of said rod of a diameter (D1) less than the width of each of said pair of plates and greater than the thickness of each of said pair of plates.

10. The apparatus according to claim 8, characterized in that at least one of said pair of plates is elongate and has a longitudinal central axis (A1) and wherein the longitudinal central axis of said rod extends coaxial with the longitudinal central axis of said one plate.

**11.** The apparatus according to claim 10, characterized in that the opening in each of said pair of plates is a slot extending in a direction parallel to the longitudinal central axis of said plate and further including a plurality of recesses (348) spaced along the opening for engaging a portion of the fastener to block movement of said plates relative to the fastener along the opening.

## Patentansprüche

**1.** Apparat (20, 120, 220) zur Fixierung der gegenseitigen Stellungen von Wirbelkörpern einer Wirbelsäule, welcher aufweist:

- eine Platte (22, 122, 222) zur Verbindung mit einem ersten Wirbelkörper, welche eine zentrale Achse (A, D, E) und erste und zweite grössere Seitenflächen (32, 34, 132, 134, 232, 234) aufweist, wobei eine der ersten und zweiten grösseren Seitenflächen dazu bestimmt ist, dem ersten Wirbelkörper gegenüberzuliegen, und wobei die genannten ersten und zweiten grösseren Seitenflächen sich im wesentlichen parallel zu einander erstrekken und durch eine Mehrzahl von kleineren Seitenflächen (36, 38, 40, 42, 136, 138, 140, 142, 236, 238, 240, 242), welche sich zwischen den genannten ersten und zweiten grösseren Seitenflächen erstrecken, miteinander verbunden sind;
- Oberflächenmittel, die eine öffnung (44, 144, 244) definieren, welche sich durch die genannte Platte zwischen den genannten ersten und zweiten grösseren Seitenflächen hindurch erstrecken, um eine Befestigungsvorrichtung (48) zur Verbindung der genannten Platte mit dem ersten Wirbelkörper aufzunehmen; gekennzeichnet durch:
- einen ersten Stab (24, 124, 224), welcher fest und nicht-freigebbar verbunden ist mit einer aus der genannten Mehrzahl von kleineren Seitenflächen der genannten Platte und sich von dieser weg erstreckt; weicher mit einem zweiten Wirbelkörper verbunden werden kann; und welcher zum Zwecke der Anpassung an eine gewünschte Krümmung eines Teils der Wirbelsäule biegbar ist; und
- einen Verbindungsteil (52, 152, 252), welcher zwischen dem genannten ersten Stab und der genannten einen kleineren Seitenfläche angeordnet ist und eine Übergangszone mit einem sich allmählich änderndem Querschnitt aufweist, um die Spannungsbeanspruchung im genannten Verbindungsteil infolge Biegung des genannten ersten Stabes möglichst klein zu halten.

**2.** Apparat nach Anspruch 1, dadurch gekennzeichnet, dass er weiter einen zweiten Stab (226) aufweist, welcher fest verbunden ist mit einer anderen aus der genannten Mehrzahl von kleineren Seitenflächen der genannten Platte und sich von dieser in einer zur zentralen Achse der genannten Platte parallelen und zum genannten ersten Stab entgegengesetzten Richtung weg erstreckt; welcher mit einem dritten Wirbelkörper verbunden werden kann; und welcher zum Zwecke der Anpassung an eine gewünschte Krümmung eines anderen Teils der Wirbelsäule biegbar ist.

**3.** Apparat nach Anspruch 1, dadurch gekennzeichnet, dass der genannte erste Stab eine zentrale Längsachse aufweist, welche in Abstand von der und parallel zu der zentralen Achse der genannten Platte verläuft; und dass eine der genannten grösseren Seitenflächen dazu bestimmt ist, dem ersten Wirbelkörper gegenüberzuliegen.

**4.** Apparat nach Anspruch 1, dadurch gekennzeichnet, dass:

- eine der genannten grösseren Seitenflächen dazu bestimmt ist, dem ersten Wirbelkörper gegenüberzuliegen;
- die genannte Platte langgestreckt ist;
- die Öffnung in der genannten Platte langgestreckt ist und sich in einer im wesentlichen zur zentralen Achse der genannten Platte parallelen Richtung erstreckt; und
- und dass die Platte weiter aufweist:
  - erste Oberflächenmittel, die eine Mehrzahl von Vertiefungen (46, 146, 246) in der genannten ersten grösseren Seitenfläche der genannten Platte definieren, welche in Abstand voneinander längs der Länge der Öffnung angeordnet sind; und
  - zweite Oberflächenmittel, die eine zweite Mehrzahl von Vertiefungen (46, 146, 246) in der genannten zweiten grösseren Seitenfläche der genannten Platte definieren, welche in Abstand voneinander längs der Länge der Öffnung angeordnet sind; wobei
    - eine der Vertiefungen der ersten und zweiten Mehrzahl von Vertiefungen dazu bestimmt ist, einen Teil der Befestigungsvorrichtung aufzunehmen, um eine Gleitbewegung der genannten Platte gegenüber der Befestigungsvorrichtung zu blockieren.

5.	Apparat nach Anspruch 1, dadurch gekennzeichnet, dass der genannte erste Stab eine zentrale Längsachse (R1) aufweist, welche koaxial zur zentralen Achse der genannten Platte verläuft.

6.	Apparat nach Anspruch 1, dadurch gekennzeichnet, dass:
   - die genannte Platte langgestreckt ist;
   - die Öffnung in der genannten Platte langgestreckt ist und sich in einer im wesentlichen zur zentralen Achse der genannten Platte parallelen Richtung erstreckt; und
   - die Platte weiter Oberflächenmittel aufweist, die eine Mehrzahl von Vertiefungen (46, 146) in der genannten zweiten grösseren Seitenfläche der genannten Platte definieren, welche in Abstand voneinander längs der Länge der Öffnung angeordnet sind; wobei
      - eine aus der Mehrzahl von Vertiefungen dazu bestimmt ist, einen Teil der Befestigungsvorrichtung aufzunehmen, um eine Gleitbewegung der genannten Platte gegenüber der Befestigungsvorrichtung zu blockieren.

7.	Apparat nach Anspruch 1, dadurch gekennzeichnet, dass er weiter Klemmschellenmittel (64, 66) zum Verbinden des genannten ersten Stabes mit dem zweiten Wirbelkörper aufweist, wobei die Klemmschellenmittel einen Teil umfassen, welcher aufweist:
   - Oberflächen, die einen Teil des genannten ersten Stabes umgeben; und
   - einen Schenkel, der sich von dem genannten Teil weg erstreckt und eine gegenüber dem Teil versetzte Öffnung zur Aufnahme einer anderen Befestigungsvorrichtung zur Verbindung der genannten Klemmschellenmittel mit dem zweiten Wirbelkörper aufweist.

8.	Apparat (320) zur Fixierung der gegenseitigen Stellungen von Wirbelkörpern einer Wirbelsäule, welcher aufweist:
   - ein Paar Platten (322, 324), welche in vorbestimmtem Abstand voneinander angeordnet sind und von denen jede mit einem separaten Wirbelkörper verbunden werden kann, wobei
      - jede Platte des Paars eine Oberfläche (332, 334) aufweist, die eine Öffnung (344) definiert, welche sich durch die Platte hindurch erstreckt und zur Aufnahme einer Befestigungsvorrichtung (346) dient, die sich in den Wirbelkörper hinein erstreckt, um die genannte Platte mit dem Wirbelkörper zu verbinden; und
   - von der Befestigungsvorrichtung unabhängige Mittel zur Verbindung der Platten des Paars miteinander, die im wesentlichen aus einem Stab (372) bestehen, welcher mit jeder Platte des genannten Paars fest und nicht-freigebbar verbunden ist, wobei der genannte Stab
      - eine Länge aufweist, welche nicht grösser als der genannte vorbestimmte Abstand ist, und
      - in mehreren Richtungen biegbar ist, um jede Platte des genannten Paars anliegend an den betreffenden Wirbelkörper zu plazieren.

9.	Apparat nach Anspruch 8, dadurch gekennzeichnet, dass:
   - jede Platte des genannten Paars eine Dicke (T1), gemessen in einer im wesentlichen zur Richtung der sich durch die Platte hindurch erstreckenden Öffnung parallelen Richtung, aufweist, welche kleiner ist als die Breite (W1) jeder Platte des Paars, gemessen im wesentlichen lotrecht zur Dicke der betreffenden Platte des Paars; und
   - der genannte Stab einen kreisförmigen Querschnitt mit einem Durchmesser (D1) aufweist, welcher, gemessen in einer auf die zentrale Längsachse (R1) lotrecht stehenden Ebene, kleiner als die Breite jeder der Platten des genannten Paars und grösser als die Dicke jeder der Platten des genannten Paars ist.

10.	Apparat nach Anspruch 8, dadurch gekennzeichnet, dass mindestens eine der Platten des Paars langgestreckt ist und eine zentrale Längsachse (A1) aufweist, wobei die zentrale Längsachse des genannten Stabes koaxial zur zentralen Längsachse der genannten einen Platte verläuft.

11.	Apparat nach Anspruch 10, dadurch gekennzeichnet, dass:
   - die Öffnung in jeder Platte des genannten Paars ein Schlitz ist, welcher sich in einer Richtung parallel zur zentralen Längsachse der genannten Platte erstreckt; und
   - er weiter eine Mehrzahl von Vertiefungen (348) aufweist, welche in Abstand voneinander längs der Öffnung angeordnet sind, um zwecks Blockierung der Bewegung der genannten Platten gegenüber der Befestigungsvorrichtung längs der Öffnung mit einem Teil der Befestigungsvorrichtung zu kuppeln.

**Revendications**

1. Dispositif (20,120,220) pour maintenir les positions relatives de corps vertébraux d'une colonne vertébrale, ledit dispositif comprenant :

   une plaque (22,122,222) pour connexion à un premier corps vertébral, ladite plaque ayant un axe central (A,D,E) et présentant une première et une deuxième grandes surfaces (32,34,132,134,232,234), une desdites première et deuxième grandes surfaces étant prévue pour être tournée vers le premier corps vertébral, lesdites première et deuxième grandes surfaces étant sensiblement parallèles l'une à l'autre et étant interconnectées par une pluralité de petites surfaces latérales (36,38,40,42,136,138,140,142,236,238,240,242) qui s'étendent entre lesdites première et deuxième grandes surfaces ;

   une configuration de surfaces définissant une ouverture (44,144,244) qui traverse ladite plaque entre lesdites première et deuxième grandes surfaces pour recevoir un élément de fixation (48) afin de relier la dite plaque au premier corps vertébral,
   caractérisé par

   une première tige (24,124,224) connectée de façon fixe et inséparable à une de ladite pluralité de petites surfaces latérales, s'étendant à partir de cette surface et étant connectable à un deuxième corps vertébral, ladite première tige pouvant être courbée afin de se conformer à une courbure désirée d'une partie de la colonne vertébrale ; et

   une région de raccordement (52,152,252)-,située entre ladite première tige et ladite petite surface latérale concernée, et ayant une zone de transition à section transversale progressivement changeante pour minimiser les contraintes qui s'exercent dans ladite région de raccordement du fait de la courbure de ladite première tige.

2. Dispositif suivant la revendication 1, caractérisé en ce qu'il comprend en outre une deuxième tige (226) reliée de façon fixe à une autre petite surface latérale de ladite plaque et s'étendant à partir de cette surface dans une direction parallèle à l'axe central de ladite plaque et à l'opposé de ladite première tige, ladite deuxième tige étant connectable à un troisième corps vertébral et pouvant être courbée afin de se conformer à une courbure désirée d'une autre partie de la colonne vertébrale.

3. Dispositif suivant la revendication 1, caractérisé en ce que ladite première tige possède un axe central longitudinal qui est espacé de l'axe central de ladite plaque et parallèle à ce dernier, et une des dites grandes surfaces est prévue pour être tournée vers le premier corps vertébral.

4. Dispositif suivant la revendication 1, caractérisé en ce qu'une desdites grandes surfaces est prévue pour être tournée vers le premier corps vertébral, ladite plaque est allongée et l'ouverture dans ladite plaque est allongée et s'étend dans une direction sensiblement parallèle à l'axe central de ladite plaque et comporte en outre des premières configurations de surface définissant une pluralité d'évidements (46,146,246) dans ladite première grande surface de ladite plaque, espacés sur la longueur de l'ouverture, et des deuxièmes configurations de surface définissant une deuxième pluralité d'évidements (46,146,246) dans ladite deuxième grande surface de ladite plaque, espacés sur la longueur de l'ouverture, un évidement desdites première et deuxième pluralités d'évidements recevant une partie de l'élément de fixation de manière à empêcher le glissement de ladite plaque par rapport à l'élément de fixation.

5. Dispositif suivant la revendication 1, caractérisé en ce que ladite première tige a un axe central longitudinal (R1) qui est coaxial à l'axe central de ladite plaque.

6. Dispositif suivant la revendication 1, caractérisé en ce que ladite plaque est allongée et l'ouverture dans ladite plaque est allongée et s'étend dans une direction sensiblement parallèle à l'axe central de ladite plaque et elle comporte en outre des configurations de surface définissant une pluralité d'évidements (46,146) dans ladite deuxième grande surface de ladite plaque, espacés sur la longueur de l'ouverture, un évidement de ladite pluralité d'évidements recevant une partie de l'élément de fixation pour empêcher le glissement de ladite plaque par rapport à l'élément de fixation.

7. Dispositif suivant la revendication 1, caractérisé en ce qu'il comprend en outre des pinces (64, 66) pour connecter ladite première tige au deuxième corps vertébral, lesdites pinces comprenant un élément présentant des surfaces qui entourent une partie de ladite première tige, et une branche s'étendant à partir dudit élément, ladite branche présentant une ouverture décalée dudit élément pour recevoir une autre fixation afin de connecter lesdites pinces au deuxième corps vertébral.

8. Dispositif (320) pour maintenir les positions relatives de corps vertébraux d'une colonne vertébrale, caractérisé en ce que ledit dispositif comprend

deux plaques (322,324) qui sont mutuellement espacées d'une distance prédéterminée, chacune de ces deux plaques étant connectable à un corps vertébral respectif ;

chacune desdites deux plaques présentant une surface (332,334) définissant une ouverture (344) qui traverse ladite plaque pour recevoir un élément de fixation (346) pénétrant dans le corps vertébral afin de connecter ladite plaque au corps vertébral ; et

des moyens indépendants de l'élément de fixation, pour interconnecter lesdites deux plaques, et consistant essentiellement en une tige (372) reliée de façon fixe et non séparable à chacune desdites deux plaques, ladite tige ayant une longueur non supérieure à ladite distance prédéterminée, ladite tige pouvant être courbée dans plusieurs directions de manière à ce que chacune desdites deux plaques soit adjacente au corps vertébral respectif.

9. Dispositif suivant la revendication 8, caractérisé en ce que chacune desdites deux plaques a une épaisseur (T1) mesurée dans une direction sensiblement parallèle à la direction d'extension de l'ouverture à travers ladite plaque, ladite épaisseur étant inférieure à une largeur (W1) de chacune desdites deux plaques mesurée sensiblement perpendiculairement à l'épaisseur d'une plaque respective desdites deux plaques, ladite tige ayant une section transversale circulaire, mesurée dans un plan perpendiculaire à l'axe central longitudinal (R1) de ladite tige, dont le diamètre (D1) est plus petit que la largeur de chacune desdites deux plaques et plus grand que l'épaisseur de chacune desdites deux plaques.

10. Dispositif suivant la revendication 8, caractérisé en ce qu'au moins une desdites deux plaques est allongée et possède un axe central longitudinal (A1), et l'axe central longitudinal de ladite tige est coaxial à l'axe central longitudinal de cette plaque.

11. Dispositif suivant la revendication 10, caractérisé en ce que l'ouverture dans chacune desdites deux plaques est une lumière s'étendant dans une direction parallèle à l'axe central longitudinal de ladite plaque et comportant en outre une pluralité d'évidements (348) espacés le long de l'ouverture pour recevoir une partie de l'élément de fixation de manière à empêcher le déplacement desdites plaques par rapport à l'élément de fixation le long de l'ouverture.

FIG.1

FIG.2

FIG. 3

FIG. 4

FIG. 8

FIG. 9

EP 0 301 489 B1

FIG.11

FIG.10

FIG.6

FIG.7

15

FIG.12

FIG.13

FIG.5

FIG.14

FIG.15

FIG.16

FIG.17

FIG.18

FIG.19

FIG.20

FIG. 21

FIG. 22

FIG.23          FIG.24